(19) **Europäisches Patentamt / European Patent Office / Office européen des brevets**

(11) **EP 3 591 614 A1**

(12) # EUROPEAN PATENT APPLICATION

(43) Date of publication:
**08.01.2020 Bulletin 2020/02**

(51) Int Cl.:
***G06T 7/12*** *(2017.01)*

(21) Application number: **18182304.8**

(22) Date of filing: **06.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Charité - Universitätsmedizin Berlin**
**10117 Berlin (DE)**
• **Beuth Hochschule Für Technik Berlin**
**13353 Berlin (DE)**

(72) Inventors:
• **GAWLIK, Kay**
**13469 Berlin (DE)**
• **KADAS, Ella Maria**
**10245 Berlin (DE)**
• **HAUßER, Frank**
**10405 Berlin (DE)**
• **BRANDT, Alexander**
**46487 Wesel (DE)**

(74) Representative: **Schulz Junghans**
**Patentanwälte PartGmbB**
**Großbeerenstraße 71**
**10963 Berlin (DE)**

(54) **METHOD AND COMPUTER PROGRAM FOR SEGMENTATION OF OPTICAL COHERENCE TOMOGRAPHY IMAGES OF THE RETINA**

(57)    The invention relates to a method and a computer program for segmentation of optical coherence tomography images of the retina comprising the steps of:

a) Acquiring image data comprising a portion of the vitreous and a portion of the retina recorded with optical coherence tomography, wherein the portion of the retina comprises at least a portion of the optical nerve head, wherein the image data comprises pixels with associated pixel values;

b) Providing a contour with a predefined initial shape and an initial position on the image data;

c) Adjusting the shape and/or the position of the contour on the image data such that the adjusted contour separates the image data in a first region comprising the vitreous and a region comprising the retina, wherein the shape and position of the contour is adjusted with an optimization method,

d) wherein the optimization method minimizes a contour-associated energy that depends on the contour shape, the contour position and the image data, wherein the contour-associated energy is minimized by adjusting the contour shape and contour position,

wherein the contour-associated energy depends on a boundary potential, wherein the boundary potential is so high in a retina portion comprised in the second region that the contour-associated energy is increased such by the boundary potential in said retina portion that the adjusted contour is located outside of said retina portion.

**Description**

The invention relates to a method and a computer program for segmentation of optical coherence tomography images of the retina.

The optic nerve head (ONH) in the human eye is the portion of the retina, where all axons from retinal ganglion cells leave the eye towards the brain, thereby forming the optic nerve.

The ONH is affected by many neurodegenerative and autoimmune inflammatory conditions. Optical coherence tomography can acquire high-resolution, three-dimensional scans of the ONH. However, the ONH's complex anatomy and pathology renders image segmentation a challenging task.

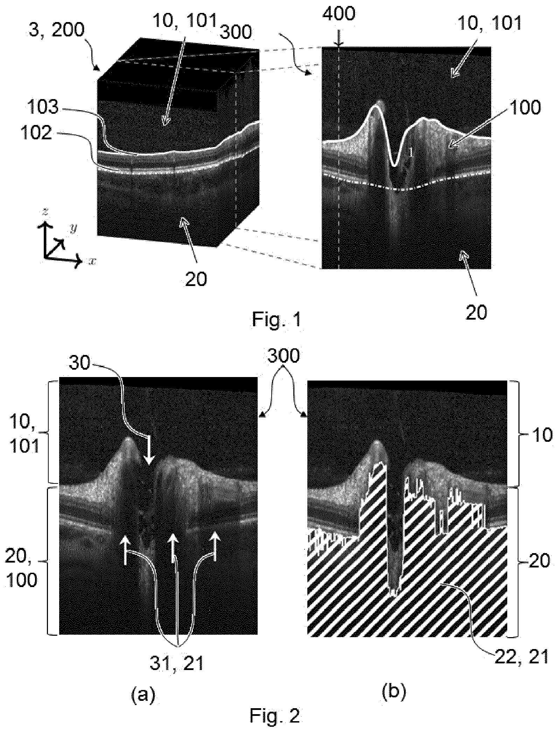

Two membranes define the ONH region and limit the ONH towards the inner and outer eye: the inner limiting membrane (ILM) and the Bruch's membrane (BM) (Fig. 1). The ILM separates the vitreous body also referred to as the vitreous from retinal tissue, while the BM is the innermost layer of the choroid, i.e. is the vascular layer of the eye. The BM is a membrane between the choroidea and the retinal pigment epithelium (RPE).

Segmenting both, the ILM and the BM, provides an important starting point for calculating imaging biomarkers of the ONH. Yet, development of suitable segmentation approaches is still an active research topic since ONH segmentation presents several difficult challenges for image analysis. In healthy ONHs, the ILM forms a cup-like recess at the center of the ONH. However, in many cases this cup-like center is formed irregularly and can even exhibit overhangs. These overhangs can cause conventional segmentation methods to generate erroneous results. Segmentation is further complicated by a dense vasculature with often loose connective tissue, which can cause ILM surfaces with vastly irregular shapes.

Furthermore, particularly optical coherence tomography scans often exhibit a low contrast representation of the ONH and a comparably low signal-to-noise ratio.

This is another reason why conventional segmentation algorithms tend to fail to correctly segment even a regular ILM progression.

Wang et al. [2] teaches a segmentation method based on a local and global intensity fitting energy functional for brain MR image (MRI) segmentation. In contrast to MRI, optical coherence tomography typically exhibits a lower contrast and a lower signal-to-noise ratio.

An object of the present invention is to provide a method and a computer program for accurate segmentation of the ILM in optical coherence tomography images. The object is achieved by the method having the features of claim 1.

Advantageous embodiments are described in the subclaims.

According to claim 1 the method for segmentation of optical coherence tomography images of the retina comprises at least the steps of:

a) Acquiring image data comprising a portion of the vitreous and a portion of the retina recorded with optical coherence tomography, wherein the portion of the retina comprises at least a portion of the optical nerve head, wherein the image data comprises pixels with associated pixel values;

b) Providing a particularly virtual contour with an associated initial shape and an initial position on, particularly relative to the image data;

c) Adjusting the shape and/or the position of the contour on the image data such that the adjusted contour particularly visually separates the image data in at least a first region comprising the vitreous and at least a second region comprising the retina, wherein the shape and position on the image data of the contour is adjusted with an, particularly iterative optimization method,

d) wherein the optimization method minimizes a contour-associated energy that depends on the contour shape, the contour position and the image data, wherein the contour-associated energy is minimized by adjusting the contour shape and contour position;

wherein the contour-associated energy depends on a boundary potential, wherein the boundary potential is so high in a particularly connected retina portion comprised in the second region that the contour-associated energy is increased so much by the boundary potential in said retina portion that the adjusted contour is located outside of said retina portion, i.e. it cannot penetrate said region.

According to the invention, the high boundary potential particularly penalizes the contour associated energy such that a different to configuration not comprising the boundary potential in the retina portion the contour-associated energy cannot be minimal within said retina portion.

The acquisition of image data is particularly achieved by recording the appropriate portion of the eye particularly by means of optical coherence tomography. Alternatively, the image data can also be re-called e.g. from a digital data storage medium or from simulated image data.

The term "image data" particularly refers to two- and/or three-dimensional image data particularly acquired by an optical coherence tomography (OCT) method.

OCT-methods particularly provide image data in form of A-scans, B-scans and/or C-scans, wherein the term "A-scan" refers to a one-dimensional line scan oriented essentially orthogonal to the retina, the term "B-scan" refers to a two-dimensional image data particularly reconstructed from laterally shifted A-scans, and the term "C-scan" particularly refers to three-dimensional image data reconstructed from a plurality of B-scans.

In the context of the specification the direction along the A-scan is particularly referred to a z-axis, while an x- and y-axis are oriented orthogonally to the z-axis in an Euclidian coordinate system.

The z-axis therefore particularly extends from the retinal tissue towards the vitreous, particularly along the optical axis of the eye.

Thus, depending on the kind of image data provided to the method of the invention, the pixels of the image data either represent the smallest area element (in case of an B-scan) or the smallest volume element (in case of a C-Scan), i.e. the pixels are voxels.

The image data can therefore be represented as two-dimensional or three-dimensional images, wherein the images are particularly grayscale images.

The pixel values particularly carry the OCT signal intensity information coded particular in form of a number.

The contour is a particularly a one- or two-dimensional manifold, i.e. a line or a surface that is to be arranged along the ILM. Once the contour is adjusted it particularly extends along the portion of the image data representing the ILM.

According to another embodiment of the invention, the image data comprises the complete nerve head and particularly not only a fraction.

According to the invention, the provided initial contour is then particularly iteratively adjusted with respect to its shape and position, until the adjusted contour separates the image data in at least a first region comprising the vitreous and at least a second region comprising the retina.

Adjusting the shape of the contour, particularly involves a change of length and/or area comprised by the shape, the contour particularly does not comprise holes.

The adjusted contour particularly represents the portion of the retina, where the ILM is located. As the ILM is represented in the image data is a line-like or area-like feature, the contour is a suitable representation of the ILM.

The optimization method is based on an energy minimization, wherein the energy is equivalent to a cost or a penalizing term and does not have to have the physical units of energy, i.e. Joule. The contour-associated energy or the associated costs or the associated penalty depends on the image data, particularly on the pixel values of the image data, as for example the intensity of the image data, the shape, for example its length or area on the image data, and/or the position.

While according to claim 1, the energy has to be minimized, it is well-known to the person skilled in the art that it is well within the scope of claim 1 if the energy is maximized, as any minimization problem can be formulated as a maximization problem.

A maximization is not an alternative embodiment to claim1 but identical to a minimization with correspondingly defined energy terms.

The approach of adjusting an energy associated with the contour provides the possibility to model a variety of shapes of the contour to fit almost any shape of the ILM. In contrast, by limiting the shape of the contour e.g. to a spline function or another predefined function, certain shapes and contours cannot be modeled with such a predefined function.

A characteristic feature of the invention is that the contour-associated energy depends on a boundary potential. A boundary potential is a cost/penalty or energy function that is configured to associate a cost/penalty or energy value to certain regions of the image data such that this region is essentially too expensive in term so cost/penalty or energy for the contour to extend in said region, as another shape of the contour that does not extend into said region would provide lower costs/penalty to the contour-associated energy. Therefore the optimization method would favor contours that are not extending in the region excluded by the boundary potential.

According to the invention, the boundary potential excludes a region in the second region comprising the retina. This excluded region is referred to as the retina portion, even though the retina portion does not comprise the entirety of the retina in the image data.

By excluding said retina portion, the contour cannot extend falsely into regions of the retinae that have a low contrast or that comprise a blood vessel. A blood vessel exhibits lower pixel values than the retinal tissue. As the pixel values in the region of a blood vessel are in the same range as the pixel values in the vitreous portion, a distinction of the a blood vessel in the retina and the vitreous is difficult. In conventional segmentation methods for the ILM, this can cause the contour to "leak" into the region comprising the blood vessel, and thus misidentifying the ILM.

The introduction of the boundary potential resolves this problem.

The boundary potential has particularly the same effect as for example removing the retina portion from the image data such that the contour cannot extend in said region.

Therefore, removing the retina portion or associating a cost/penalty to it that prohibits penetration of the contour in said region is identical and therefore well within the scope of the claimed invention.

While the method is configured for image data generated by optical coherence tomography, it is particularly suitable also for image data derived from other imaging modalities such as MRI, or X-ray tomography.

According to another embodiment of the invention, the contour, particularly the adjusted contour is displayed on a display particularly together with the image data or a selected portion of the image data.

In some cases it can be useful to analyze the shape of the contour such that is it is sufficient to solely display the contour.

Together with the contour also contour specific parameters can be displayed, such as the surface area or line length of the contour and/or contour height.

Alternatively, the contour is displayed together with at least portion of the image data such that visual assessment of the segmentation quality is possible.

Also, here additional contour and thus ILM specific parameters can be displayed on the display.

According to another embodiment of the invention, the contour is adjusted such that it approximates, particularly coincides with the inner limiting membrane in the image data.

As stated above, the ILM is particularly represented by a line or an area-like structure (B-scan or C-scan) in the image data having particularly higher pixel values than the surrounding tissue, a line-like or area-like contour is well-suited for segmenting the image data in a first region comprising the vitreous, i.e. the region "above" the ILM and the first region "below" the ILM, i.e. the retinal tissue.

According to another embodiment of the invention, the boundary potential has a first level with a first value and a second level with a second value, wherein in the retina portion the boundary potential assumes the second value and outside the retina portion the boundary potential assumes the first value.

The values of the boundary potential can be associated with each pixel of the image. The first value is particularly zero and thus does not affect the shape of the contour in the region where the boundary potential assumes its first value. The second value is a positive value or infinity high enough to prevent the contour to comprise pixels of the image data associated with the second value of the boundary potential.

According to another embodiment of the invention, the boundary potential is a step function, wherein the step is at a boundary of the retina portion.

According to this embodiment, the boundary potential particularly does not assume other levels except the first and second level, i.e. the boundary potential forms a "hard" barrier between the retina portion and the region extending outside the retina portion.

Therefore, the boundary potential, particularly the step, extends particularly along a delimiting boundary of the retina portion.

A step function particularly comprises at least one step, i.e. a discontinuity between two values of the step function, where not first derivative of the step function exists for the step function.

The step function is particularly a binary function that assumes only two values throughout the image data.

This embodiment allows for an accurate exclusion of the retina portion, i.e. an accurate exclusion of blood vessels.

According to another embodiment of the invention, the image data comprises at least one B-scan, particularly consisting of a plurality of A-scans, wherein the at least one B-scan has the pixels arranged in a N x M matrix comprising M columns, particularly each extending along the A-Scan direction, and N rows of pixels, wherein the retina and the vitreous are oriented such with respect to the matrix that the columns extend from a lower end of the second region towards an upper end of the first region, particularly wherein the rows of the image data extend essentially along the Bruch-membrane comprised by the retina.

The term "lower end of the second region" particularly refers to the portion of the second region that is closer to the border of the image data than an "upper end" of the second region that is located closer towards the vitreous.

In the same manner the term "upper end of the first region" can be understood, namely that the upper end of the first region particularly refers to a portion of the image data that is located closer to the border of the image data in comparison the a "lower end of the first region" that is particularly located closer to the retinae tissue, i.e. the second region.

The term" lower end" and "upper end" particularly refer to locations that particularly differ with respect to the position on the z-axis of the image data, wherein the z-axis particularly extends along a direction extending form the retinae tissue towards the vitreous, particularly wherein the z-axis is orthogonal to the Bruch-membrane.

According to another embodiment of the invention, for each B-scan and for each column of the B-Scan, the pixel values in the column are normalized to a predefined maximum pixel value, e.g. the pixel value 1 and particularly to a minimum pixel value, e.g. zero, such that the image data is normalized column wise.

According to another embodiment of the invention, wherein for each, particularly normalized column - starting from the lower end - the boundary potential for each pixel of the column is set to the second value until the pixel value of a pixel in the respective column exceeds a predefined threshold value, particularly wherein the pixel value exceeds the predefined value for the first time, particularly wherein the threshold value is 45% of a highest pixel value of all pixel values in the respective column, wherein, when the pixel value exceeds the predefined threshold value, the boundary potential is set to the first value in the respective column.

When the image data comprises normalized pixel values for each column, the predefined maximum value is particularly 0.45 times the maximum pixel value.

This embodiment allows for an accurate definition of the retina portion, as, starting from the retinal tissue, i.e. the lower end of the second region, the method identifies an increase of the pixel intensities, wherein, the retina portion is limited by pixel values that exceed the predefined pixel value. In order to limit the retina portion correctly the retina portion is limited when the first pixel value of the pixels in the respective column exceeds said predefined maximum value.

This embodiment allows for a column-wise processing of the image data in order to generate the retina portion.

The resulting boundary potential is therefore particularly a binary potential in form of a step function in each column of the B-scan and/or the C-scan.

Method according to one of the preceding claims, wherein the contour-associated energy $F$ depends on the boundary potential $V(x)$ according to

$$F = F^{other} + F^{bound} = F^{other} + \int_{\Omega_1} V(\mathrm{x})d\mathrm{x},$$

wherein $F$ is the contour-associated energy, $F^{other}$ are other energy terms contributing to the contour-associated energy, $\Omega_1$ is the first region, $V(x)$ is the boundary potential and x is the location particularly the pixel in the image data.

It is noted that, while the image data consists of discrete pixels the formula for the contour-associated energy is formulated for continuous data x, it is obvious to the person skilled in the art how to adapt the formula to the case of discrete pixels and therefore to the case of discrete locations x. in the image data, such that the formula can be used for discrete image data. For example the integral $\int_{\Omega_1} V(x)dx$ is replaced by a sum over all pixels comprised in the first region $\Omega_1 : \sum_{x_i \in \Omega_1} V(x_i)$.

According to another embodiment of the invention, the contour-associated energy $F$ further depends on a global and a local energy, a surface energy and a volume energy, particularly wherein the other energy terms comprise at least one of the global, the local, the surface energy and/or the volume energy.

In the context of the specification a global energy $F^{gif}$ particularly accounts for the mean squared fluctuation of the pixel values of the first region and the second region from a predefined, constant value, wherein the fluctuations are particularly centred around the mean values of the first and second region respectively, which is the predefined constant value. The global energy is particularly designed to produce a "force" that arranged the contour such in the image that the variance of the pixel values in each region is identical.

In the context of the specification a local energy $F^{lif}$ particularly accounts for local intensity changes such that particularly edges, i.e. rapid changes in the pixel values can be identified independent of a non-constant background.

A surface energy particularly accounts for the surface area, or line length of the contour. The surface energy is particularly designed to penalize contours with a large area (3D-image data with a two-dimensional contour) or long lines (2D-image data with a one-dimensional contour) respectively.

The surface energy therefore acts as a pulling force on the contour that pulls the contour in order to keep the surface area of the contour small or the line length of the contour short.

In the context of the specification the volume energy particularly accounts for size of the first region, wherein the volume energy grows with the size, e.g. with the volume of the first region, i.e. the volume energy particularly acts as a force that keeps the first region small.

According to another embodiment of the invention, the other energy terms are given by: $F^{other} = \omega F^{gif} + (1 - \omega)F^{lif} + \mu F^{surf} + v F^{vol}$,
wherein $\omega, \mu, v$ are pre-factors, wherein

$$F^{gif} = \lambda_1 \int_{\Omega_1} |I(\mathrm{x}) - c_1|^2 d\mathrm{x} + \lambda_2 \int_{\Omega_2} |I(\mathrm{x}) - c_2|^2 d\mathrm{x},$$

wherein $F^{gif}$ is a global energy with $c_1$, $c_2$ as well as $\lambda_1$, $\lambda_2$ being pre-factors, $I(x)$ representing the pixel value at position x in the image data, and $\Omega_1$, $\Omega_2$ being the first and the second region, wherein

$$F^{lif} = \lambda_1 \int \int_{\Omega_1} K_\sigma\,(\mathrm{x}-\mathrm{y})|I(\mathrm{y})-f_1(\mathrm{x})|^2 d\mathrm{x}d\mathrm{y}\ +$$

$$\lambda_2 \int \int_{\Omega_2} K_\sigma\,(\mathrm{x}-\mathrm{y})|I(\mathrm{y})-f_2(\mathrm{x})|^2 d\mathrm{x}d\mathrm{y}$$

wherein $F^{lif}$ is a local energy, with $\lambda_1$, $\lambda_2$ being pre-factors, particularly the same as the ones in the global energy, x, $y$ are particularly three-dimensional positions in the image data, $K_\sigma$ being a compact support kernel, with a kernel size of $\sigma$, $f_1(\mathrm{x})$, $f_2(\mathrm{x})$ representing fit functions configured to locally approximate the pixel value $I(\mathrm{x})$ in $\Omega_1$ and $\Omega_2$, respectively.

$$F^{surf} = \int_C\ d\mathrm{s},$$

wherein $F^{surf}$ is a surface energy that accounts for the surface area of the contour $C$,

$$F^{vol} = \int_{\Omega_1} 1 d\mathrm{x}$$

wherein $F^{vol}$ is a volume energy, calculated from the volume comprised by the first region $\Omega_1$.

It is noted that the expressions $d\mathrm{x}$, $d\mathrm{y}$ and $d\mathrm{s}$ refer to the infinitesimal volume or area elements respectively.

As stated already above, as the image data comprises pixels and thus the coordinates of the image data are not continuous, the integrals have to reformulated to reflect a the discrete image data. The given formulas are therefore to be understood as a general formulation for the energies, the translation to the discrete case is known to the person skilled in the art.

According to another embodiment of the invention, for each column the pre-factors $c_1$ and $c_2$ are adjusted such that they can vary across the columns, wherein the pre-factors are adjusted for each column according to

$$c_1(m) = \frac{c_1}{2}\Big(1 - max\big(I(\mathrm{x}_m)\big)\Big)\ \text{and}\ c_2(m) = \frac{c_2}{2}\Big(1 - max\big(I(\mathrm{x}_m)\big)\Big),$$

wherein $max$ is the maximum operator and $m$ is the $m^{th}$ column.

This embodiment allows for a better estimate of the global energy. A similar effect could be achieved by adjusting the pixel values in each column, however adjusting the pixel values would also affect the local energy, wherein adjusting the pre-factors leaves the local energy unaffected.

Adjusting the pre-factors allows accounting for regions where the pixels comprising the retinal tissue have a low intensity and comparably little contrast with respect to the pixels comprising vitreous.

According to another embodiment of the invention, the Bruch's membrane in the retina is identified and a second contour is generated extending along the Bruch's membrane, wherein the contour and/or the image data are adjusted for the shape of the second contour and thus the Bruch's membrane.
This embodiment allows a unified comparison of image data acquired from different patients, form different eyes and or at different time points.

By referencing the shape of the contour to the Bruch's membrane a solid reference is provided, as particularly in many diseases the Bruch membrane remains unaffected, where the ONH changes its shape. This change of shape can then be quantified by means of the adjustment of the contour, representing the ILM, to the Bruch's membrane.

The Bruch's membrane can be identified by a variety of segmentation methods.

According to another embodiment of the invention, a transformation is applied to the contour and/or to the image data, wherein said transformation is configured to level the second contour planar, wherein the transformed contour and/or the transformed image data is displayed, particularly either separately or as an overlay.

From the transformed contour a variety of shape parameters of the ILM and thus the ONH can be derived in a unified manner, such that the comparability of different data sets is achieved.

According to another embodiment of the invention, a distance between the contour and the second contour is determined, wherein the distance is determined for each point of the contour to a respective point of the second membrane, wherein for each point of the contour the distance is displayed or plotted particularly two-dimensionally, particularly wherein from the distance of the contour to the second contour a contour height relative to the second contour is determined.

The points of the contour are particularly coordinates of the contour. As the contour can be adjusted by the method according to the invention such that sub-pixel accuracy is achieved with respect to the segmentation of the retina, the distance is determined from the point or coordinates of the contour and the second contour in order to sustain the segmentation accuracy.

Determining the distance between the contour and the second contour allows for a unified and comparable determination of the height of the contour and thus other parameters such an ONH-depth, an ONH-width and the like.

According to another aspect of the invention, the problem is solved by a computer program comprising instructions which, when the program is executed by a computer, cause the computer to carry out the method according to the invention.

According to another aspect of the invention, the problem is solved by a computer program product comprising instructions which, when the program is executed by a computer, cause the computer to carry out the method according to the invention.

The term 'computer', or system thereof, is used herein as ordinary context of the art, such as a general purpose processor or a micro-processor, RISC processor, or DSP, possibly comprising additional elements such as memory or communication ports. Optionally or additionally, the term 'computer' or derivatives thereof denotes an apparatus that is capable of carrying out a provided or an incorporated program and/or is capable of controlling and/or accessing data storage apparatus and/or other apparatus such as input and output ports. The terms 'processor' or 'computer' particularly denote also a plurality of processors or computers connected, and/or linked and/or otherwise communicating, possibly sharing one or more other resources such as a memory.

Figure description

In the following exemplary embodiments are disclosed by means of a detailed figure description. It is shown in

Fig. 1    a volume scan (C-scan) and a B-scan of the ONH, with the contour and the second contour overlaid on the image data;

Fig. 2    a B-scan with the boundary potential;

Fig. 3    a comparison between the conventional method and the method according to the invention;

Fig. 4    Pre-processing steps for estimating an initial contour;

Fig. 5    Another comparison between the method according to the invention and the state of the art; and

Fig. 6    surface plot of the contour as derived from OCT image data according to the invention.

Segmentation of the ONH is often incorrect, particularly of the ONH comprises overhangs in the cup-like portion. As a consequence, a tedious and time consuming manual ILM segmentation correction is necessary before ONH shape parameters can be derived in scans with deep ONH cupping or steep forms.

For example in Fig. 1 a conventional segmentation method for the ILM 103 and its result is shown. The contour segmenting the ILM 103 is depicted as a solid white line, wherein the second contour extending along the Bruch's membrane 102 is shown as a broken white line. As can be seen, the method fails to accurately identify the boundary between the retinal tissue 100 and the vitreous 101.

In the left panel of Fig. 1 a volume scan, also referred to as C-scan 200, of an optical coherent tomography method is shown and on the right panel a B-scan 300 along a section from the C-scan 200 as indicated by the dotted lines is shown . Each B-scan 300 in turn consists of a plurality of A-scans 400 as indicated by the arrow.

In the B-scan 300 the ONH 104 is visible and its cup-like shape. The upper dark region is the vitreous 101 of the eye, and is also referred to as the first region 10 in the context of the specification. The vitreous 101 is delimited by the ILM 103 and the retinal tissue 100 that generally exhibits higher gray values and is referred to as the second region 20.

The three-arrows marked up with x,y,z depict the orientation of the axis of a coordinate system.

The method according the invention achieves an accurate segmentation particularly with a modified Chan-Vese (CV) based segmentation method with sub-pixel accuracy that is fast, robust and able to correctly detect ILM 103 surfaces regardless of ONH 104 shape complexity or overhangs 105. Key features of the method according to the invention are:

- ILM 103 overhangs 105, which are frequently seen in eyes from patients with neurologic or autoimmune neuroinflammatory diseases can be segmented correctly.

- A lower boundary constraint, also referred to as boundary potential 22 is introduced in the Chan-Vese method in order to avoid the adjusted contour 1 to leak in tissue regions with low contrast.
- The pre-factors $c_1$ and $c_2$ are obtained as a result of an optimization method and are further adjusted after several iteration steps by a scaling factor that incorporates the data locally. This greatly increased the segmentation accuracy.

Methods

Region based Active Contour methods

Active contours have been introduced by [3] as powerful methods for image segmentation. A contour $C$ is moved over the image data 3, where the dynamics are defined by the gradient flow of some suitable energy functional $F = F(C)$. Here, $F(C)$ depends on the intensity function $I = I(x) \in [0,1]$ of the given gray scale image data 3. Thus, the final segmentation is obtained by finding the energy minimizing contour $C$ for the given image data $I$. In region-based methods, two regions defined and separated by the contour $C$ are used to model the energy function $F$.

Let $\Omega$ denote the image domain. In the context of OCT volumes (optical coherence tomography volumes), $\Omega$ is a three-dimensional volume, and $C$ is a two-dimensional surface contour 1 that divides the retinal tissue 100 from the vitreous body 101, i.e. the adjusted contour is the segmented ILM 103, satisfying:

$$inside(C) = \Omega_1 \cong retinal\ tissue; \quad outside(C) = \Omega_2 \cong vitreous\ body$$

The classical CV model [1] approximates the intensity function $I(x)$ by some piecewise constant function with values $c_1$ and $c_2$ in $\Omega_1$ and $\Omega_2$, respectively, x= (x, y, z) is a voxel in the image $I$. The energy $F^{CV}$ is defined as the weighted sum of a region based global intensity fitting (gif) energy $F^{gif}$, penalizing deviations of $I(x)$ from the corresponding value $c_1$ or $c_2$ respectively, a surface energy $F^{surf}$ given by the surface area of $C$, and a volume energy $F^{vol}$ given by the volume of $\Omega_1$ (also referred to a first region 10):

$$F^{CV} = F^{gif} + \mu F^{surf} + \nu F^{vol},$$

with

$$F^{gif} = \lambda_1 \int_{\Omega_1} |I(x) - c_1|^2 dx + \lambda_2 \int_{\Omega_2} |I(x) - c_2|^2 dx$$

$$F^{surf} = \int_C ds$$

$$F^{vol} = \int_{\Omega_1} 1 dx$$

Note that by minimizing $F^{CV}$, the surface energy $F^{surf}$ leads to a smooth contour surface $C$, whereas the volume energy $F^{vol}$ yields a balloon force. Moreover, minimizing $F^{CV}$ with respect to the values of $c_1$ and $c_2$ results in choosing $c_1$ and $c_2$ as the average intensities in the first and second region $\Omega_1$ and $\Omega_2$ (also referred to the second region 20), respectively. The positive weighting parameters $\lambda_1$, $\lambda_2$ control the influence of the corresponding energy term. Finding good values for these parameters is crucial for obtaining the desired results. Usually the parameters $\lambda_1$, $\lambda_2$ are taken to be equal and may therefore be set to $\lambda_1 = \lambda_2 = 1$.

Challenges in OCT images

The classical CV model [1] is robust in segmenting noisy images, without clearly defined boundaries. Moreover, complicated shapes such as overhangs, various connected components, even topological changes are handled naturally. However, applying the original formulation of CV to OCT scans does not yield good results. As already discussed in the literature, see e.g. [2], CV fails to provide good segmentation if the two delineated regions $\Omega_1$ and $\Omega_2$ are strongly non-uniform in their gray values (i.e. pixel values). Performance gets even worse in the presence of very dark regions inside the tissue, see Fig. 2(a), where a slice (B-scan 300) of a typical OCT volume scan is depicted, or in regions with extreme high intensities inside the tissue 100 or the vitreous 101. As a consequence, using local image averages, as proposed in the classical CV model, is not able to provide a satisfactory segmentation.

Arrow 30 shows the ONH region with no retinal layer information except some tissue remaining from nerve fibers and the ILM 103; the upwards-pointing arrows 31 show shadows caused by the presence of large blood vessels.

In Fig. 2(b) the boundary potential 22 in the retina portion 22 comprised by the retinal tissue 100 is depicted as a hatched area.

In the following, this problem is addressed by adapting the global fitting energy to a local one in the narrow band setting [2] and by using a boundary potential 22 to prevent the contour 1 to move into certain regions, particularly in a retina portion 21 comprised by the second region 20. These modifications result in a very stable segmentation method for OCT scans.

Global fitting energy

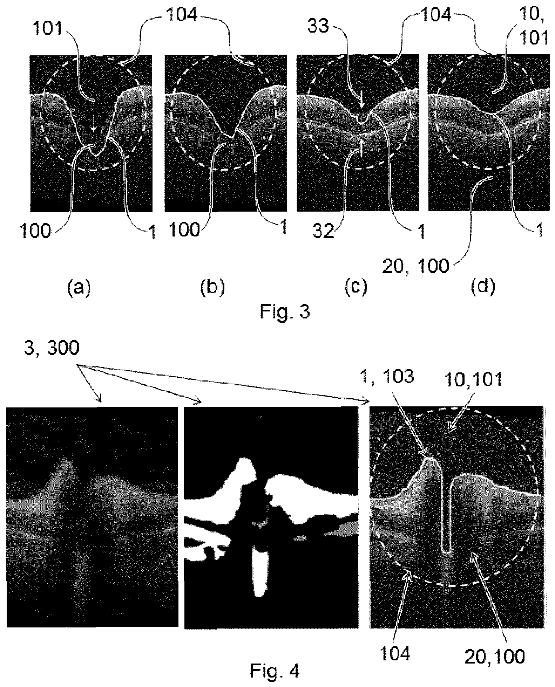

The values $c_1$ and $c_2$, obtained after optimization are adjusted column-wise (per A-scan 400) in order to obtain correct segmentation results at the ONH 104, where the tissue 100 has very low intensity and little contrast to the vitreous 101, see Fig. 3(a) white arrow. Fig. 3(a) shows an example of an ONH 104 (indicated by a white circle) with very low contrast (indicated by a white arrow) compared to the vitreous 101. This leads to the contour 1 leaking into the tissue 100.

According to the invention, the values $c_1$ and $c_2$ are adjusted for each column using the formula:

$$c_1(m) = \frac{c_1}{2}\left(1 - max\left(I(\mathrm{x}_m)\right)\right) \text{ and } c_2(m) = \frac{c_2}{2}\left(1 - max\left(I(\mathrm{x}_m)\right)\right)$$

m denoting the $m^{th}$ column of a B-scan 300. This significantly improves the segmentation results, see e.g. Fig. 3(b). Fig. 3(b) shows the same scan as shown in Fig. 3(a) with correctly detected ILM 103 after scaling the values $c_1$ and $c_2$ according to the invention. Additionally, in order to prevent the contour to penetrate the retina in regions with dark upper but hyperintense lower layers, see e.g. Fig. 3(c), these values are rescaled again, after the interface has almost reached the desired ILM contour. Thus, in Fig. 3(c) an example of an ONH 104 region with low intensity values at the inner layers (white arrow 32) compared to the dark vitreous 101 as well as to the hyperintense outer layers (white arrow 33). This would cause the contour 1 to falsely detect parts of the lower layers as vitreous 101. The same scan with correctly detected ILM by rescaling values c1 and c2 is shown in Fig. 3(d).

The factor used is computed with the same formula as above, but the maximum intensity considers only voxels from the top of the volume to 77 μm (20 px) below the current interface position. Segmentation results obtained after this scaling step are shown in Fig. 3(d).

By rescaling $c_1$ and $c_2$ instead of rescaling the column intensities the local fitting energy $F^{lif}$ remains unaffected (for the definition see below).

Boundary potential

To prevent the contour $C$, at the ONH 104, from evolving into dark areas 21, caused by absence of retinal layers and presence of large vessels, characteristic to this region (see e.g. Fig. 2(a)), a particularly local boundary potential $V(\mathrm{x})$ (also referred to with the reference numeral 22) is introduced:

$$F^{bound} = \int_{\Omega_1} V(\mathrm{x}) d\mathrm{x}$$

This potential 22 is set to a very high value $\rho$ at these dark regions 21 that are detected as follows: in each column, starting from bottom to top, $V(\mathrm{x})$ is set to $\rho$ until the first pixel value $I(\mathrm{x})$ is larger than 45% of the maximum pixel value in that column. All the other voxels are set to zero, see Fig. 2(b) for an example. These dark regions 21 are also referred to as the retina portion 21 in the specification.

Local intensity fitting Energy

In images with large intensity inhomogeneities, e.g. caused by varying illumination, it is not sufficient to minimize only a global intensity fitting energy. In order to achieve better segmentation results, two fitting functions $f_1(\mathrm{x})$ and $f_2(\mathrm{x})$ are introduced, which are configured to locally approximate the intensity $I(\mathrm{x})$ in $\Omega_1$ and $\Omega_1$, respectively. The local intensity fitting energy functional is defined as:

$$F^{lif} = \lambda_1 \int \int_{\Omega_1} K_\sigma(\text{x} - \text{y})|I(\text{y}) - f_1(\text{x})|^2 d\text{x}d\text{y}$$

$$+ \lambda_2 \int \int_{\Omega_2} K_\sigma(\text{x} - \text{y})|I(\text{y}) - f_2(\text{x})|^2 d\text{x}d\text{y}$$

Similar to the pre-factors $c_1$ and $c_2$ explicit formulas for functions $f_1(x)$ and $f_2(x)$ are obtained by energy minimization [2]. Since all calculations are restricted to a narrow band along the contour 1, a compact support kernel for $K_\sigma$ is used based on a binomial distribution with $\sigma$ representing the kernel size. Note that these modifications also considerably reduce the computation time as compared to a global convolution.

The energy model

In a final step, the influence of the local and global intensity fitting energy, are combined similar to the work presented in [2] by introducing another weight parameter $\omega$ and to arrive at the functional $F$ according to the invention:

$$F = \omega F^{gif} + (1 - \omega)F^{lif} + \mu F^{surf} + \nu F^{vol} + F^{bound}$$

Implementation

Initial contour

For initialization a basic two-dimensional segmentation algorithm is used to create the initial contour (start segmentation). In a first step, morphological filters (erosion and subsequent dilation with 15 x 7 ellipse structure element) and a smoothing filter (Gaussian blur with kernel size 15 x 7 and variance $\sigma_x$ = 6, $\sigma_z$ = 3 are used to reduce speckle noise. In the next step, each pixel with at least 35% of the maximum column intensity is set to white, i.e. to 1. The remaining pixels are set to black, i.e. 0. To keep only the tissue connected to the retina, enhanced at the previous step, the pixel values of all connected components consisting of less than 2,400 pixels, which corresponds to 0.11 mm$^2$ in the used OCT image data set, are set to gray, e.g. 0.5. Finally, in each column of the image data, the contour is set at the first white pixel from top to bottom. If no white pixel exists, the first gray pixel is taken instead. These processing steps are exemplified in Fig. 4. In Fig. 4 the three processing steps to obtain initial segmentation are shown. Fig. 4 (a) shows filtering of the image data with a morphological and a Gauss filter. Fig. 4 (b) shows thresholding, and neglecting small connected components, and Fig. 4(c) shows the initial contour 1.
The OCT image size was is particularly 384 x 496 x 145 voxels.

Parameter optimization

An automatic parameter optimization procedure was used to find values for the parameters $\omega$, $v$, $c_1$ and $c_2$.
Presented OCT image data consisted of 3D ONH scans obtained with a spectral-domain OCT (Heidelberg Spectralis SDOCT, Heidelberg Engineering, Germany) using a custom ONH scan protocol with 145 B-scans, focusing the ONH with a scanning angle of 15° x 15° and a resolution of 384 A-scans per B-scan. The spatial resolution in x-direction is approximately 12.6 $\mu$m, in axial direction approximately 3.9 $\mu$m and the distance between two B-scans is approximately 33:5 $\mu$m. The database consists of 416 ONH volume scans that capture a wide spectrum of ONH topological changes specific to neuroinflammatory disorders (71 healthy control eyes, 31 eyes affected by idiopathic intracranial hypertension, 60 eyes from neuromyelitis optical spectrum disorders, and 252 eyes of multiple sclerosis patients). 140 scans were randomly from this database, which presented different characteristics, from scans with good quality up to noisy ones, from healthy but also eyes from patients with different neurological disorders, in order to cover a broad range of shapes.
40 ONH scans were manually segmented and used as ground truth for the optimization process. These will be called the group40. The remaining 100 scans - in the following referred to as the group100 - were used for the validation of the segmentation results and assessment of image quality influence on the segmentation results. Incomplete volume scans as well as those with retinas damaged by other pathologies were not included.

Error measurement

All 40 scans of the group40 were manually segmented and checked by an experienced grader. From this dataset, twenty images were used for optimization, while the other twenty for validating the results. For one optimization run, ten files were randomly chosen from the optimization set. The measure used for the minimization process was defined as the sum of the errors for the parameter $\omega$, $v$, $c_1$ and $c_2$. An error metric similar to the one described in [4] was employed, where the error is defined as the number of wrongly assigned voxels, i.e. the sum of the number of false positive and false negative. Note that this metric does not depend on the position of the retina. In order to compare different optimization results, the accumulated error of all the twenty scans of the optimization set was used.

Optimization algorithm

The method chosen is the multi-space optimization differential evolution algorithm as provided by GNU Octave [5].

This algorithm creates a starting population of 40 individuals with random values. In our case, an individual represents a parameter set for $\omega$, $v$, $c_1$ and $c_2$ together with the accumulated segmentation error of the 10 selected volume scans. During optimization, the algorithm crosses and mutates the individuals to create new ones, and drops out newly created or old ones depending on which exhibits larger errors. We allowed for at most 2000 iterations and set box constraints for all four parameters. Note that for each newly created individual, the cost function (error) has to be evaluated by first performing 10 segmentations for the randomly chosen OCT-scans, then calculating the error by comparing with results from manual segmentation. Thus, each iteration step is computationally demanding. The differential evolution algorithm has been chosen since it is derivative free and supports the setting of specific bounds for the parameters. Moreover, we observed a high reproducibility of the finally obtained optimal parameter set. To perform the optimization, we used the Docker Swarm on OpenStack infrastructure from [6], which allowed to do parallel computations on a PC cluster.

Results

Optimization results

The parameters given in the first line, namely, and $v$= 0.03248; $\omega$ = 0.15915; $c_1$= 0.24206; $c_2$= 0.94945 have been chosen for all subsequent calculations. Note that the four parameters are not independent from each other as it can be seen in the definition of the energy functional. The parameter that shows the largest variation is the balloon force weight parameter $\omega$, $v$, $c_1$ and $c_2$. This variation is highly influenced by the presence or absence of one specific volume scan in the randomly chosen optimization set (subset of 10 out of 20), which appears as outlier with highest error in all 10 error distributions. This occurs because the parameters will account for this particular scan if it is contained in the optimization set.

Results:

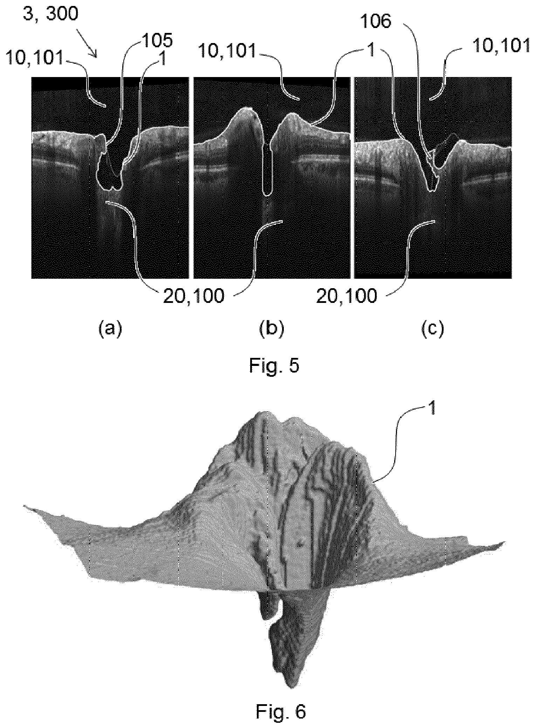

In Fig. 5 results from the method according to the invention are shown. The white solid line depicts the adjusted contour 1 as obtained by the method of the invention, and the broken line depicts the segmentation as obtained by a conventional segmentation method. The conventional segmentation method shows significant deviations from the true extend of the ILM 103. In Fig. 5(a) the conventional method fails to identify the slight overhang 105 of the ILM 103, while in Fig. 5(b) the conventional method short-cuts the comparably deep cup-like region of the ONH and in Fig. 5(c) the conventional method does not identify a protrusion 106 of the ILM 103 into the vitreous 101. The method according to the invention does identify all these features correctly.

In Fig. 6 a three-dimensional representation of the two-dimensional adjusted contour 1 is shown.

References

[1] T. F. Chan and L. A. Vese, "Active contours without edges." IEEE Trans. Image Process. 10, 266-277 (2001).

[2] L. Wang, C. Li, Q.-S. Sun, D.-S. Xia, and C.-Y. Kao, "Active contours driven by local and global intensity fitting energy with application to brain MR image segmentation." Comp. Med. Imag. Graph. 33, 520-531 (2009).

[3] M. Kass, A. Witkin, and D. Terzopoulos, "Snakes: Active contour models," International Journal of Computer Vision 1, 321-331 (1988).

[4] A. A. Taha and A. Hanbury, "Metrics for evaluating 3d medical image segmentation: analysis, selection, and tool," BMC Med. Imaging 15, 29 (2015).

[5] S. Das, A. Abraham, U. K. Chakraborty, and A. Konar, "Differential Evolution Using a Neighborhood-Based Mutation Operator." IEEE Trans. Evol. Comput. 13, 526-553 (2009).

[6] C. Jansen, M. Witt, and D. Krefting, Employing Docker Swarm on OpenStack for Biomedical Analysis (Springer International Publishing, Cham, 2016), p. 303-318.

**Claims**

1.  A method for segmentation of optical coherence tomography images of the retina comprising the steps of:

    a) Acquiring image data (3) comprising a portion of the vitreous (101) and a portion of the retina (100) recorded with optical coherence tomography, wherein the portion of the retina (100) comprises at least a portion of the optical nerve head (104), wherein the image data (3) comprises pixels with associated pixel values;
    b) Providing a contour (1) with a predefined initial shape and an initial position on the image data (3);
    c) Adjusting the shape and/or the position of the contour (1) on the image data (3) such that the adjusted contour (1) separates the image data (3) in a first region (10) comprising the vitreous (101) and a second region (20) comprising the retina (100), wherein the shape and position of the contour (1) is adjusted with an optimization method,
    d) wherein the optimization method minimizes a contour-associated energy that depends on the contour shape, the contour position and the image data (3), wherein the contour-associated energy is minimized by adjusting the contour shape and contour position;

    **characterized in that** the contour-associated energy depends on a boundary potential (22), wherein the boundary potential (22) is so high in a retina portion (21) comprised in the second region (100) that the contour-associated energy is increased such by the boundary potential (22) in said retina portion (21) that the adjusted contour (1) is located outside of said retina portion (22).

2.  Method according to claim 1, wherein the contour (1) is adjusted such that it coincides with the inner limiting membrane (103) in the image data (3).

3.  Method according to claim 1 or 2, wherein the boundary potential (22) has a first level with a first value and a second level with a second value, wherein in the retina portion (21) the boundary potential (22) assumes the second value and outside the retina portion (21) the boundary potential (22) assumes the first value.

4.  Method according to one of the preceding claim, wherein the boundary potential (22) is a step function, wherein the step of the step function is at a boundary of the retina portion (21).

5.  Method according to one of the preceding claims, wherein the image data (3) comprises at least one B-scan (300), wherein the at least one B-scan (300) has the pixels arranged in a matrix $N \times M$ comprising $M$ columns and $N$ rows, wherein the retina (100) and the vitreous (101) are oriented such with respect to the matrix that the columns extend from a lower end of the second region (20) towards an upper end of the first region (10), particularly wherein the rows of the image data (3) extend essentially along the Bruch-membrane (102) comprised by the retina (100).

6.  Method according to claim 5, wherein for each column - starting from the lower end - the boundary potential (22) for each pixel of the column is set to the second value until the pixel value in the column exceeds a predefined threshold value, particularly wherein the threshold value is 45% of a maximum pixel value in the respective column, wherein, when the pixel value exceeds the predefined threshold value, the boundary potential (22) is set to the first value in the respective column.

7.  Method according to one of the preceding claims, wherein the contour-associated energy $F$ depends on the boundary potential $V(x)$ (22) according to

$$F = F^{other} + F^{bound} = F^{other} + \int_{\Omega_1} V(\mathrm{x})dx,$$

wherein F is the contour-associated energy, $F^{other}$ are other energy terms contributing to the contour-associated energy, $\Omega_1$ is the first region and $V(\mathrm{x})$ is the boundary potential (22).

**8.** Method according to one of the preceding claims, wherein the contour-associated energy $F$ further depends on a global and a local energy, a surface energy and a volume energy, particularly wherein the other energy terms comprise at least one of the global, the local, the surface energy and/or the volume energy.

**9.** Method according to claim 7 or 8, wherein the other energy terms are given by:

$$F^{other} = \omega F^{gif} + (1 - \omega)F^{lif} + \mu F^{surf} + \nu F^{vol},$$

wherein $\omega$, $\mu$, $v$ are pre-factors, wherein

$$F^{gif} = \lambda_1 \int_{\Omega_1} |I(\mathrm{x}) - c_1|^2 dx + \lambda_2 \int_{\Omega_2} |I(\mathrm{x}) - c_2|^2 dx,$$

Wherein $F^{gif}$ is a global energy with $c_1$, $c_2$ as well as $\lambda_1$, $\lambda_2$ being pre-factors, $I(\mathrm{x})$ representing the pixel value at position x in the image data, and $\Omega_1$, $\Omega_2$ being the first and the second region,

$$F^{lif} = \lambda_1 \int \int_{\Omega_1} K_\sigma (\mathrm{x} - \mathrm{y})|I(\mathrm{y}) - f_1(\mathrm{x})|^2 dx dy +$$

$$\lambda_2 \int \int_{\Omega_2} K_\sigma (\mathrm{x} - \mathrm{y})|I(\mathrm{y}) - f_2(\mathrm{x})|^2 dx dy$$

wherein $F^{lif}$ is a local energy, with $\lambda_1$, $\lambda_2$ being pre-factors, x, y are coordinates in the image data, $K_\sigma$ being a compact support kernel, with a kernel size of $\sigma$, $f_1(\mathrm{x})$, $f_2(\mathrm{x})$ representing fit functions configured to locally approximate the pixel value $I(\mathrm{x})$.

$$F^{surf} = \int_C ds,$$

wherein $F^{surf}$ is a surface energy that accounts for the surface area of the contour $C$,

$$F^{vol} = \int_{\Omega_1} 1 dx,$$

wherein $F^{vol}$ is a volume energy, calculated from the volume comprised by the first region $\Omega_1$.

**10.** Method according to claim 9, wherein for each column the pre-factors $c_1$ and $c_2$ are adjusted such that they can vary across the columns, wherein the pre-factors are adjusted for each column according to

$$c_1(m) = \frac{c_1}{2}\left(1 - max\big(I(\mathrm{x}_m)\big)\right) \text{ and } c_2(m) = \frac{c_2}{2}\left(1 - max\big(I(\mathrm{x}_m)\big)\right),$$

wherein $max$ is the maximum operator and $m$ is the $m^{th}$ column.

**11.** Method according to claim 1, wherein the Bruch's membrane (102) in the retina (100) is identified and particularly a second contour is generated extending along the Bruch's membrane (100), wherein the contour (1) and/or the image data (3) is adjusted for the shape of the second contour.

**12.** Method according to claim 11, wherein a transformation is applied to the contour (1) and/or to the image data (3) that is configured to level the second contour planar, wherein the transformed contour (1) and/or the transformed image data (3) is displayed.

13. Method according to one of the claims 11 to 12, wherein a distance between the contour (1) and the second contour is determined, wherein the distance is determined for each section of the contour (1) to a respective section of the second membrane, wherein for each section of the contour (1) the distance is displayed or plotted particularly two-dimensionally, particularly wherein from the distance of the contour (1) to the second contour a contour height relative to the second contour is determined.

14. A computer program comprising instructions which, when the program is executed by a computer, cause the computer to carry out the method of claim 1.

Fig. 1

(a)                              (b)

Fig. 2

Fig. 3

Fig. 4

Fig. 5

Fig. 6

Europäisches
Patentamt

European
Patent Office

Office européen
des brevets

## EUROPEAN SEARCH REPORT

Application Number

EP 18 18 2304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GONZÁLEZ-LÓPEZ ANA ET AL: "A web-based framework for anatomical assessment of the retina using OCT", BIOSYSTEMS ENGINEERING, ACADEMIC PRESS, UK, vol. 138, 23 April 2015 (2015-04-23), pages 44-58, XP029274745, ISSN: 1537-5110, DOI: 10.1016/J.BIOSYSTEMSENG.2015.04.001 | 1-5, 7-11,13, 14 | INV. G06T7/12 |
| Y | * abstract * | 12 | |
| A | * Section 3.1. Layer Segmentation Service; page 47; figures 2, 3 * * Sub-section 3.1.1 Preprocessing; page 47, right-hand column * * Sub-section 3.1.2 Active contour model-based segmentation; page 47, right-hand column - page 49, right-hand column; figures 4-7 * * Sub-section 3.2.2. Extracting statistical indicators; page 52; figure 13 * * Sub-section 4.1 Layer segmentation validation; page 53, right-hand column - page 54, right-hand column * | 6 | |
| Y | US 2017/132793 A1 (HU QINGMAO [CN] ET AL) 11 May 2017 (2017-05-11) * abstract * * paragraph [0099] - paragraph [0102] * | 12 | TECHNICAL FIELDS SEARCHED (IPC) G06T |

-/--

The present search report has been drawn up for all claims

| Place of search | Date of completion of the search | Examiner |
|---|---|---|
| Munich | 5 December 2018 | Rusu, Alexandru |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 18 2304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Azadeh Yazdanpanah: "AN ACTIVE CONTOUR APPROACH FOR SEGMENTATION OF INTRA-RETINAL LAYERS IN OPTICAL COHERENCE TOMOGRAPHY IMAGES", , 9 December 2010 (2010-12-09), pages 1-105, XP055530257, Retrieved from the Internet: URL:http://summit.sfu.ca/system/files/irit ems1/12154/etd6407_AYazdanPanahGoharRizi.p df [retrieved on 2018-12-05] * abstract * * Section 3. Active contours methods for image segmentation; page 22 - page 35 * * Section 4. Multi-phase Segmentation: Active contour without edge plus shape and weight approach; page 36 - page 49 * * Section 4.5 Results; page 51 - page 65 * ----- | 1-14 | |
| A | US 2017/119242 A1 (JIA YALI [US] ET AL) 4 May 2017 (2017-05-04) * abstract * * paragraph [0022] * * paragraph [0067] * * paragraph [0084] - paragraph [0085] * ----- -/-- | 1-14 | TECHNICAL FIELDS SEARCHED (IPC) |

The present search report has been drawn up for all claims

| Place of search | Date of completion of the search | Examiner |
|---|---|---|
| Munich | 5 December 2018 | Rusu, Alexandru |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 18 18 2304

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GHOLAMI PEYMAN ET AL: "Intra-retinal segmentation of optical coherence tomography images using active contours with a dynamic programming initialization and an adaptive weighting strategy", PROGRESS IN BIOMEDICAL OPTICS AND IMAGING, SPIE - INTERNATIONAL SOCIETY FOR OPTICAL ENGINEERING, BELLINGHAM, WA, US, vol. 10483, 14 February 2018 (2018-02-14), pages 104832M-104832M, XP060100672, ISSN: 1605-7422, DOI: 10.1117/12.2292095 ISBN: 978-1-5106-0027-0 * the whole document * | 1-14 | |
| A | MUJAT MIRCEA ET AL: "Retinal nerve fiber layer thickness map determined from optical coherence tomography images", OPTICS EXPRESS, OSA PUBLISHING, US, vol. 13, no. 23, 14 November 2005 (2005-11-14), pages 9480-9491, XP002583846, ISSN: 1094-4087 * the whole document * | 1-14 | |

TECHNICAL FIELDS
SEARCHED (IPC)

The present search report has been drawn up for all claims

| Place of search | Date of completion of the search | Examiner |
|---|---|---|
| Munich | 5 December 2018 | Rusu, Alexandru |

EPO FORM 1503 03.82 (P04C01)

page 3 of 3

## ANNEX TO THE EUROPEAN SEARCH REPORT
## ON EUROPEAN PATENT APPLICATION NO.

EP 18 18 2304

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2018

| Patent document cited in search report | | Publication date | Patent family member(s) | | Publication date |
|---|---|---|---|---|---|
| US 2017132793 | A1 | 11-05-2017 | CN | 106485721 A | 08-03-2017 |
| | | | US | 2017132793 A1 | 11-05-2017 |
| | | | WO | 2017036231 A1 | 09-03-2017 |
| US 2017119242 | A1 | 04-05-2017 | NONE | | |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. F. CHAN ; L. A. VESE.** Active contours without edges. *IEEE Trans. Image Process,* 2001, vol. 10, 266-277
- **L. WANG ; C. LI ; Q.-S. SUN ; D.-S. XIA ; C.-Y. KAO.** Active contours driven by local and global intensity fitting energy with application to brain MR image segmentation. *Comp. Med. Imag. Graph.,* 2009, vol. 33, 520-531
- **M. KASS ; A. WITKIN ; D. TERZOPOULOS.** Snakes: Active contour models. *International Journal of Computer Vision,* 1988, vol. 1, 321-331
- **A. A. TAHA ; A. HANBURY.** Metrics for evaluating 3d medical image segmentation: analysis, selection, and tool. *BMC Med. Imaging,* 2015, vol. 15, 29
- **S. DAS ; A. ABRAHAM ; U. K. CHAKRABORTY ; A. KONAR.** Differential Evolution Using a Neighborhood-Based Mutation Operator. *IEEE Trans. Evol. Comput.,* 2009, vol. 13, 526-553
- **C. JANSEN ; M. WITT ; D. KREFTING.** Employing Docker Swarm on OpenStack for Biomedical Analysis. Springer International Publishing, 2016, 303-318